Europäisches Patentamt

(19) European Patent Office

Office européen des brevets

(11) Numéro de publication : **0 138 705**
**B1**

(12) **FASCICULE DE BREVET EUROPÉEN**

(45) Date de publication du fascicule du brevet :
15.03.89

(51) Int. Cl.⁴ : **A 61 K 7/16**

(21) Numéro de dépôt : **84402026.3**

(22) Date de dépôt : **09.10.84**

(54) Composition destinée à l'hygiène et à la santé du parodonte des gencives et des dents.

(30) Priorité : 13.10.83 FR 8316284

(43) Date de publication de la demande :
24.04.85 Bulletin 85/17

(45) Mention de la délivrance du brevet :
15.03.89 Bulletin 89/11

(84) Etats contractants désignés :
AT BE CH DE GB IT LI LU NL SE

(56) Documents cités :
FR--A-- 2 420 970
US--A-- 3 932 604
US--A-- 3 970 747
US--A-- 4 198 394

(73) Titulaire : **Goupil, Jean-Jacques**
**30 Avenue du Président Wilson**
**F-94230 CACHAN (FR)**

(72) Inventeur : **Goupil, Jean-Jacques**
**30 Avenue du Président Wilson**
**F-94230 CACHAN (FR)**

(74) Mandataire : **Petit, Hélène**
**Cabinet Hélène Petit 94, Avenue Kléber**
**F-75116 Paris (FR)**

Jouve, 18, rue St-Denis, 75001 Paris, France

## Description

La présente invention a pour objet une composition destinée à l'hygiène et à la santé du parodonte des gencives et dents et en particulier utile dans la prévention de la carie dentaire.

Cette composition peut se présenter sous forme de pâte ou de gel et contient une association de sels fluorés et d'un édulcorant le xylitol.

Le xylitol a déjà été introduit dans des compositions dentifrices diverses à son titre d'édulcorant non cariogène.

Ainsi, le brevet US A 3970747 décrit un dentifrice qui contient un agent sucrant humectant non cariogène, le xylitol. Il est par ailleurs précisé dans ce brevet que la composition dentifrice peut contenir, si désiré, des ingrédients additionnels tels que des composés fluorés. Toutefois, dans une composition de ce genre, l'action anti-carie est seulement liée à la proportion de sels fluorés présente, alors que le xylitol joue un rôle d'édulcorants sucrés humectants et non cariogènes.

Selon la présente invention, il est apparu de façon imprévisible que certaines associations de sels fluorés avec le xylitol possédaient une activité anti-carie considérablement supérieure à celle que l'on pouvait normalement espérer en liaison avec la présence des sels fluorés pour une concentration déterminée. On a alors pu mettre en évidence l'action synergique du xylitol sur l'activité anti-carie des sels fluorés, au sein de certaines associations.

La présente invention a pour objet des compositions pour l'hygiène et la santé du parodonte des gencives et des dents, se présentant sous forme de pâte, de gel ou toute autre forme convenable, caractérisées en ce qu'elles contiennent une association de deux seuls fluorés, le monofluorophosphate de sodium associé au fluorure de sodium, le bifluorure d'ammonium associé au fluorure de sodium, ladite association présentant une concentration en ion fluor comprise entre 200 mg et 2 000 mg pour 100 g de composition, c'est-à-dire comprise entre 2 000 et 20 000 ppm, concentration en ion fluor de préférence comprise entre 250 et 1 000 g pour 100 g de composition, soit entre 2 500 et 10 000 ppm, du xylitol à une concentration de 5 à 50 % en poids par rapport à la composition finale, et de préférence comprise entre 10 et 25 %, au sein d'un excipient convenable.

Les sels fluorés dont l'activité anti-carie est connue peuvent être le fluorure de sodium, le monofluorophosphate de sodium, le bifluorure d'ammonium, le fluorure de manganèse ou le fluorure de potassium. L'association de ces sels entre eux peut se faire en diverses proportions assurant qu'au total la teneur en ion fluor soit au moins égale à 200 mg. Une des associations préférées de la présente invention est l'association de monofluorophosphate de sodium et de fluorure de sodium.

Les compositions suivantes sont données à titre illustratif et non limitatif de la présente invention :

Composition n° 1 à 250 mg en ion fluor

Principes actifs :

| | |
|---|---|
| Monofluorophosphate de sodium | 0,7600 g % |
| Fluorure de sodium | 0,3315 g % |
| Xylitol | 20,0000 g % |

Excipient :
Sodium carraghénate
Sodium phosphate monoacide
Sodium hexamétaphosphate
Silice précipitée
Silice de Neubourg
Titane oxyde    } q.s.p. 100 g
Méthyl paraoxybenzoate
Sodium saccharinate
Huiles essentielles
Sorbitol à 70 %
Eau

Dans cette composition 100 mg d'ion fluor (soit 1 000 ppm) sont apportés par le monofluorophosphate et 150 mg d'ion fluor (soit 1 500 ppm) sont apportés par le fluorure de sodium.

Composition n° 2 à 250 mg en ion fluor

Principes actifs :

| | |
|---|---|
| Monofluorophosphate de sodium | 0,7600 g % |
| Fluorure de sodium | 0,3315 g % |
| Xylitol | 15,0000 g % |

2

Excipient :
Sodium carraghénate
Sodium phosphate monoacide
Sodium hexamétaphosphate
Silice précipitée
Silice de Neubourg
Titane oxyde                        } q.s.p. 100 g
Méthyl paraoxybenzoate
Sodium saccharinate
Huiles essentielles
Sorbitol à 70 %
Eau

Composition n° 3 à 250 mg en ion fluor

Principes actifs :

| | |
|---|---|
| Monofluorophosphate de sodium | 0,8000 g % |
| Fluorure de sodium | 0,3200 g % |
| Xylitol | 10,0000 g % |

L'excipient est le même que celui de la composition n° 1.

Composition n° 4 à 250 mg en ion fluor

Principes actifs :

| | |
|---|---|
| Bifluorure d'ammonium | 0,2260 g % |
| Fluorure de sodium | 0,2210 g % |
| Xylitol | 10,0000 g % |

Dans cette composition 150 mg d'ion fluor (soit 1 500 ppm) sont apportés par le bifluorure d'ammonium, et 100 mg d'ion fluor (soit 1 000 ppm) sont apportés par le fluorure de sodium.
L'excipient est le même que celui de la composition n° 1.

Composition n° 5 à 250 mg en ion fluor

Principes actifs :

| | |
|---|---|
| Bifluorure d'ammonium | 0,1500 g % |
| Fluorure de sodium | 0,3310 g % |
| Xylitol | 15,0000 g % |

Dans cette composition 100 mg d'ion fluor (soit 1 000 ppm) sont apportés par le bifluorure d'ammonium, et 150 mg d'ion fluor (soit 1 500 ppm) sont apportés par le fluorure de sodium.
L'excipient est le même que celui de la composition n° 1.

Composition n° 6 à 250 mg en ion fluor

Principes actifs :

| | |
|---|---|
| Bi-fluorure d'ammonium | 0,2250 g % |
| Fluorure de sodium | 0,2210 g % |
| Xylitol | 20,0000 g % |

Dans cette composition 150 mg d'ion fluor (soit 1 500 ppm) sont apportés par le bi-fluorure d'ammonium, et 100 mg d'ion fluor (soit 1 000 ppm) sont apportés par le fluorure de sodium.
L'excipient est le même que celui de la composition n° 1.

Composition n° 7 à 250 mg en ion fluor

Principes actifs :

| | |
|---|---|
| Bi-fluorure d'ammonium | 0,1500 g % |
| Fluorure de sodium | 0,3310 g % |
| Xylitol | 20,0000 g % |

# EP 0 138 705 B1

L'excipient est le même que celui de la composition n° 1.

## Composition n° 8 à 250 mg en ion fluor

Principes actifs :

| | |
|---|---|
| Bi-fluorure d'ammonium | 0,2250 g % |
| Fluorure de sodium | 0.2210 g % |
| Xylitol | 15,0000 g % |

L'excipient est le même que celui de la composition n° 1.

## Composition n° 9 à 500 mg en ion fluor

Principes actifs :

| | |
|---|---|
| Monofluorophosphate de sodium | 1,5200 g % |
| Fluorure de sodium | 0,6630 g % |
| Xylitol | 20,0000 g % |

Dans cette composition 200 mg d'ion fluor (soit 2 000 ppm) sont apportés par le monofluorophosphate de sodium, et 300 mg d'ion fluor (soit 3 000 ppm) sont apportés par le fluorure de sodium.
L'excipient est le même que celui de la composition n° 1.

## Composition n° 10 à 500 mg en ion fluor

Principes actifs :

| | |
|---|---|
| Monofluorophosphate de sodium | 0,8000 g % |
| Fluorure de sodium | 0,8730 g % |
| Xylitol | 15,0000 g % |

L'excipient est le même que celui de la composition n° 1.

## Composition n° 11 à 500 mg en ion fluor

Principes actifs :

| | |
|---|---|
| Bi-fluorure d'ammonium | 0,4500 g % |
| Fluorure de sodium | 0,4420 g % |
| Xylitol | 20,0000 g % |

Dans cette composition 300 mg d'ion fluor (soit 3 000 ppm) sont apportés par le bi-fluorure d'ammonium, et 200 mg d'ion fluor (soit 2 000 ppm) sont apportés par le fluorure de sodium.
L'excipient est le même que celui de la composition n° 1.

## Composition n° 12 à 500 mg en ion fluor

Principes actifs :

| | |
|---|---|
| Bi-fluorure d'ammonium | 0,4500 g % |
| Fluorure de sodium | 0,4420 g % |
| Xylitol | 15,0000 g % |

L'excipient est le même que celui de la composition n° 1.

## Composition n° 13 à 500 mg en ion fluor

Principes actifs :

| | |
|---|---|
| Monofluorophosphate de sodium | 1.1360 g % |
| Fluorure de sodium | 0,7730 g % |
| Xylitol | 15,0000 g % |

On peut préparer les compositions selon l'invention selon le mode opératoire suivant.
On utilise un mélangeur disperseur par exemple pour une unité de 1 500 KG.
On réalise une préparation préalable :
— Dans une cuve en inox de 200 litres, on dissout dans 30 litres d'eau purifiée les fluorures, on fait

4

passer par pompage dans une autre cuve en inox réservée à cet usage. Cette solution constitue la Solution A.

— Dans la cuve de 200 litres, on dissout dans 100 litres d'eau purifiée sous agitation électrique : la Saccharinate de Sodium et le Benzoate de Sodium : Solution B.

Dans le disperseur :

— On introduit l'eau purifiée restante, le Sorbitol, le Xylitol, les Phosphates, l'Oxyde de Titane, la Silice Précipitée.

— On met l'appareil en marche et on laisse tourner le temps nécessaire pour obtenir un mélange homogène.

— On fait arriver par pompage la solution B en la filtrant.

— On ajoute le Lauryl Sulfate de Sodium, et quelques minutes après, le Carraghénate de Sodium, la Solution A, enfin l'aromatiseur dans lequel on aura dissout au préalable sous électro-agitation le Paraoxybenzoate de méthyle acide.

— On laisse tourner l'appareil pendant 30 minutes jusqu'à parfaite homogénéité.

— On désaère sous vide durant 15 minutes.

— On filtre ensuite la pâte et on la passe sur homogénéisateur et on stocke en cuve.

L'activité anti-carie des compositions selon l'invention a été mise en évidence par des essais cliniques comparatifs conduits entre les compositions de l'invention contenant des sels fluorés et du xylitol, et des compositions témoins contenant seulement des sels fluorés.

1er Essai

Cet essai a porté sur la composition selon l'invention contenant 250 mg en ion fluor et du xylitol sur quatre compositions témoins contenant respectivement 125 mg en ion fluor, 250 mg en ion fluor, 500 mg en ion fluor, 1 000 mg en ion fluor.

Composition selon l'invention

Principes actifs :

| | |
|---|---|
| Monofluorophosphate de sodium | 0,7600 g % |
| Fluorure de sodium | 0,3315 g % |
| Xylitol | 20,0000 g % |

Excipient :
Sodium phosphate monoacide ⎫
Sodium hexamétaphosphate ⎪
Sodium carraghénate ⎪
Silice précipitée ⎪
Silice de Neubourg ⎪
Titane oxyde ⎬ q.s.p. 100 g
Méthyl paraoxybenzoate ⎪
Sodium saccharinate ⎪
Huiles essentielles ⎪
Sorbitol à 70 % ⎪
Eau ⎭

Composition témoin n° 1 à 125 mg en ion fluor

Principes actifs :

| | |
|---|---|
| Monofluorophosphate de sodium | 0,380 g % |
| Fluorure de sodium | 0,165 g % |

Excipient :
Sodium phosphate monoacide ⎫
Sodium Hexamétaphosphate ⎪
Sodium carraghénate ⎪
Silice précipitée ⎪
Silice de Neubourg ⎪
Titane oxyde ⎬ q.s.p. 100 g
Methyl paraoxybenzoate ⎪
Sodium Saccharinate ⎪
Huiles essentielles ⎪
Sorbitol à 70 % ⎪
Eau ⎭

5

Composition témoin n° 2 à 250 mg en ion fluor

Principes actifs :

| | |
|---|---|
| Monofluorophosphate de sodium | 0,7600 g % |
| Fluorure de sodium | 0.3315 g % |

Excipient :
Sodium phosphate monoacide
Sodium hexamétaphosphate
Silice précipitée
Silice de Neubourg
Titane oxyde
Méthyl paraoxybenzoate ⟩ q.s.p. 100 g
Sodium carraghénate
Huiles essentielles
Sorbitol à 70 %
Eau

Composition témoin n° 3 à 500 mg en ion fluor

Principes actifs :

| | |
|---|---|
| Monofluorophosphate de sodium | 0,8000 g % |
| Fluorure de sodium | 0,8730 g % |

L'excipient est le même que celui de la composition témoin n° 2.

Composition témoin n° 4 à 1 000 mg en ion fluor

Principes actifs :

| | |
|---|---|
| Monofluorophosphate de sodium | 1,136 g % |
| Fluorure de sodium | 1,878 g % |

L'excipient est le même que celui de la composition témoin n° 2.

La composition selon l'invention à 250 mg en ion fluor, et 20 % en xylitol, a été testée, en même temps que les quatre compositions témoins, sur des patients porteurs de caries, à raison de deux brossages par jour de 2 minutes 1/2 chacun, l'un le matin après le petit déjeuner et l'autre le soir au coucher.

L'étude a porté sur une population d'enfants de 8 à 14 ans, vivant pendant l'année scolaire en internat afin de pouvoir assurer des contrôles journaliers, et a duré trois années consécutives. Des chirurgiens dentistes ont assuré périodiquement les contrôles des caries selon les critères définis et utilisés par l'O.M.S., à savoir la mesure de l'indice CAO dents : C = carie, A = absente, O = obturée.

La différence entre l'indice CAO dents en fin de test et l'indice CAO dents au bilan initial donne l'incrément. Il ressort de cette définition qu'à un incrément élevé correspond une augmentation sensible du nombre des caries en fin de test par rapport au nombre des caries au bilan initial. Au contraire, à un incrément faible correspond une augmentation faible du nombre des caries en fin de test par rapport au nombre des caries au bilan initial.

Les résultats cliniques de l'expérimentation ont été rassemblés dans le tableau suivant.

Tableau des résultats cliniques - Essai n° 1

Incrément = indice CAO Dents en fin de test - CAO Dents au bilan initial

| Tranche d'Age | Incréments | | | | |
|---|---|---|---|---|---|
| | témoin n° 1 à 125 mg en ion fluor | témoin n° 2 à 250 mg en ion fluor | témoin n° 3 à 500 mg en ion fluor | témoin n° 4 à 1000 mg en ion fluor | Composition à 250mg en ion fluor et 20% xylitol |
| 8 - 11 ans | 2,61 | 1,68 | - | - | 0,95 |
| 9 - 12 ans | 3,14 | 0,72 | 1 | 0,63 | 0,98 |
| 10 - 13 ans | 3,94 | 2,67 | 1,13 | 0,78 | 1,07 |
| 11 - 14 ans | - | - | 2,87 | 0,89 | - |
| Tous âges confondus | 2,80 | 1,50 | 1,25 | 0,76 | 1 |

EP 0 138 705 B1

Il apparaît directement à la lecture de ce tableau que les incréments obtenus pour la composition de· l'invention à 250 mg en ion fluor et à 20 % de xylitol sont inférieurs aux incréments obtenus pour les trois premiers témoins, et supérieurs aux incréments obtenus pour le témoin n° 4 correspondant à une composition à 1 000 mg en ion fluor. Ceci signifie que la composition selon l'invention a une capacité de réduction des caries supérieure à la capacité de réduction des compositions témoins contenant respectivement 125 mg en ion fluor, 250 mg en ion fluor, et 500 mg en ion fluor. Ceci démontre que la présence de xylitol dans la composition de l'invention à 250 mg en ion fluor rend cette composition plus active dans la réduction des caries que la composition correspondante à 250 mg en ion fluor et sans xylitol, et également plus active que la composition à 500 mg en ion fluor sans xylitol.

Ceci peut également être exprimé en pourcentage. Ainsi, par rapport au groupe témoin à 125 mg, la composition selon l'invention à 250 mg en ion fluor et à 20 % de xylitol entraîne une réduction des caries mesurée par la différence des incréments correspondants, soit :

$$2,80 - 1 = 1,80$$

ce qui peut s'exprimer en pourcentage :

$$\frac{1,80 \times 100}{2,80} = 64 \%$$

La composition selon l'invention entraîne une réduction des caries de 64 % par rapport à la composition témoin à 125 mg en ion fluor.

De même par rapport au groupe témoin à 250 mg la composition selon l'invention à 250 mg en ion fluor et à 20 % de xylitol entraîne une réduction des caries de 32 %

$$\left( \frac{1,5 - 1}{1,5} \times 100 = 32 \% \right) \cdot$$

Par rapport aux groupes témoins à 500 mg, la composition selon l'invention à 250 mg en ion fluor et à 20 % de xylitol entraîne une réduction des caries de 20 %

$$\left( \frac{1,25 - 1}{1,25} \times 100 = 20 \% \right) \cdot$$

On peut également examiner le pourcentage de réduction des caries en comparant les groupes suivants par rapport au premier groupe témoin à 125 mg en ion fluor :

Groupe à 250 mg en ion fluor : réduction des caries de 46 %
Groupe à 500 mg en ion fluor : réduction des caries de 56 %
Groupe à 250 mg en ion fluor + 20 % de xylitol : réduction des caries de 64 %
Groupe à 1 000 mg en ion fluor : réduction des caries de 76 %.

Il apparaît là encore clairement que la composition selon l'invention à 250 mg en ion fluor et 20 % de xylitol a une activité de réduction des caries supérieure à celle des compositions à 250 mg en ion fluor seul et à 500 mg en ion fluor seul.

On constate que l'introduction de xylitol dans la formule à 250 mg en ion fluor apporte une amélioration de la réduction des caries de 18 % (64 %-46 %).

2e Essai

Cet essai a porté sur une composition selon l'invention contenant 500 mg en ion fluor et du xylitol et sur trois compositions témoins contenant respectivement 250 mg en ion fluor, 500 mg en ion fluor, 1 000 mg en ion fluor.

Composition selon l'invention

Principes actifs :

| | |
|---|---|
| Monofluorophosphate de sodium | 0,8000 g % |
| Fluorure de sodium | 0,8730 g % |
| Xylitol | 20,0000 g % |

L'excipient était le même que l'excipient décrit pour la composition n° 1 de l'invention.

8

# EP 0 138 705 B1

Composition témoin n° 1 à 250 mg en ion fluor

Principes actifs :

Monofluorophosphate de sodium ............................................................. 0,7600 g %
Fluorure de sodium ............................................................................... 0.3315 g %

L'excipient est le même que celui de la composition n° 1 selon l'invention.

Composition témoin n° 2 à 500 mg en ion fluor

Principes actifs :

Monofluorophosphate de sodium ............................................................. 0,8000 g %
Fluorure de sodium ............................................................................... 0,8730 g %

L'excipient est le même que celui de la composition n° 1 de l'invention.

Composition témoin n° 3 à 1 000 mg en ion fluor

Monofluorophosphate de sodium ............................................................. 1,136 g %
Fluorure de sodium ............................................................................... 1,878 g %

L'excipient est le même que celui de la composition n° 1 de l'invention.
Les résultats cliniques d'expérimentation de l'essai n° 2 ont été rassemblés dans le tableau suivant :

(Voir tableau page 10)

9

Tableau des résultats cliniques - Essai n° 2

| Tranche d'âge | Incréments | | | |
|---|---|---|---|---|
| | témoin n° 1 à 250 mg en ion fluor | témoin n° 2 à 500 mg en ion fluor | témoin n° 3 à 1000 mg en ion fluor | Composition à 500mg en ion fluor + 20 % xylitol |
| 9 - 12 ans | 0,72 | 0,77 | 0,62 | 0,75 |
| 10 - 13 ans | 0,81 | 0,92 | 0,73 | 0,87 |
| 11 - 14 ans | 2,67 | 1,55 | 0,82 | 0,63 |
| Tous âges confondus | 1,4 | 1,10 | 0,72 | 0,75 |

Il apparaît directement à la lecture de ce tableau que la composition selon l'invention à 500 mg en ion fluor et 20 % de xylitol possède des incréments inférieurs aux incréments des deux compositions témoins à 250 mg en ion fluor et à 500 mg en ion fluor, et du même ordre de grandeur que les incréments de la composition témoin n° 3 à 1 000 mg en ion fluor.

Ceci signifie que l'activité de réduction de caries de la composition selon l'invention est plus grande que l'activité des compositions témoins à 250 et 500 mg en ion fluor, et équivalente à l'activité de la composition témoin à 1 000 mg en ion fluor.

Par rapport à la composition témoin à 250 mg seul, la composition de l'invention à 500 mg en ion fluor et 20 % de xylitol entraîne une réduction de caries de 46 %

$$\left( \frac{1,4 - 0,75}{1,4} \times 100 = 46\,\% \right)$$

Par rapport à la composition témoin à 500 mg en ion fluor, la composition de l'invention entraîne une réduction de caries de 32 %

$$\left( \frac{1,10 - 0,75}{1,10} \times 100 = 32\,\% \right).$$

**Revendications**

1. Compositions pour l'hygiène et la santé du parodonte des gencives et des dents, se présentant sous forme de pâte, de gel ou toute autre forme convenable, caractérisées en ce qu'elles contiennent une association de deux sels fluorés, le monofluorophosphate de sodium associé au fluorure de sodium, le bi-fluorure d'ammonium associé au fluorure de sodium, ladite association présentant une concentration en ion fluor comprise entre 200 mg et 2 000 mg pour 100 g de composition, c'est-à-dire comprise entre 2 000 et 20 000 ppm, concentration en ion fluor de préférence comprise entre 250 et 1 000 mg pour 100 g de composition, soit entre 2 500 et 10 000 ppm, du xylitol à une concentration de 5 à 50 % en poids par rapport à la composition finale, et de préférence comprise entre 10 et 25 % au sein d'un excipient convenable.

2. Compositions pour l'hygiène et la santé du parodonte des gencives et des dents, se présentant sous forme de pâte, de gel ou toute autre forme convenable, selon la revendication 1, caractérisées en ce qu'elles contiennent une association de monofluorophosphate de sodium et de fluorure de sodium présentant une concentration en ion fluor de 250 mg pour 100 g de composition et du xylitol à la concentration de 20 % en poids par rapport à la composition finale, au sein d'un excipient convenable.

3. Compositions pour l'hygiène et la santé du parodonte des gencives et des dents, se présentant sous forme de pâte, de gel ou toute autre forme convenable, selon la revendication 1, caractérisées en ce qu'elles contiennent une association de monofluorophosphate de sodium et de fluorure de sodium présentant une concentration en ion fluor de 250 mg pour 100 g de composition et du xylitol à la concentration de 15 % en poids par rapport à la composition finale, au sein d'un excipient convenable.

4. Compositions pour l'hygiène et la santé du parodonte des gencives et des dents, se présentant sous forme de pâte, de gel ou toute autre forme convenable, selon la revendication 1, caractérisées en ce qu'elles contiennent une association de monofluorophosphate de sodium et de fluorure de sodium présentant une concentration en ion fluor de 250 mg pour 100 g de composition et du xylitol à la concentration de 10 % en poids par rapport à la composition finale, au sein d'un excipient convenable.

5. Composition pour l'hygiène et la santé du parodonte des gencives et des dents, se présentant sous forme de pâte, de gel ou toute autre forme convenable, caractérisée en ce qu'elle contient 0,76 % de monofluorophosphate de sodium, 0,33 % de fluorure de sodium, 20 % de xylitol, au sein d'un excipient convenable.

6. Compositions pour l'hygiène et la santé du parodonte des gencives et des dents, se présentant sous forme de pâte, de gel ou toute autre forme convenable, selon la revendication 1, caractérisées en ce qu'elles contiennent une association de bi-fluorure d'ammonium et de fluorure de sodium présentant une concentration en ion fluor de 250 mg pour 100 g de composition et du xylitol à la concentration de 20 % en poids par rapport à la composition finale, au sein d'un excipient convenable.

7. Compositions pour l'hygiène et la santé du parodonte des gencives et des dents, se présentant sous forme de pâte, de gel ou toute autre forme convenable, selon la revendication 1, caractérisées en ce qu'elles contiennent une association de bi-fluorure d'ammonium et de fluorure de sodium présentant une concentration en ion fluor de 250 mg pour 100 g de composition et du xylitol à la concentration de 15 % en poids par rapport à la composition finale, au sein d'un excipient convenable.

8. Compositions pour l'hygiène et la santé du parodonte des gencives et des dents, se présentant sous forme de pâte, de gel ou toute autre forme convenable, selon la revendication 1, caractérisées en ce qu'elles contiennent une association de monofluorophosphate de sodium et de fluorure de sodium présentant une concentration en ion de 500 mg pour 100 g de composition et du xylitol à une concentration de 20 % en poids par rapport à la composition finale, au sein d'un excipient convenable.

9. Composition pour l'hygiène et la santé du parodonte des gencives et des dents, se présentant sous forme de pâte, de gel ou toute autre forme convenable, selon la revendication 8, caractérisée en ce qu'elle contient une association de 0,800 % de monofluorophosphate de sodium, et 0,873 % de fluorure de sodium, 20 % de xylitol au sein d'un excipient convenable.

10. Compositions pour l'hygiène et la santé du parodonte des gencives et des dents, se présentant sous forme de pâte, de gel ou toute autre forme convenable, selon la revendication 1, caractérisées en ce qu'elles contiennent une association de monofluorophosphate de sodium et de fluorure de sodium présentant une concentration en ion de 500 mg pour 100 g de composition et du xylitol à une concentration de 15 % en poids par rapport à la composition finale, au sein d'un excipient convenable.

11. Composition pour l'hygiène et la santé du parodonte des gencives et des dents, se présentant sous forme de pâte, de gel ou toute autre forme convenable, selon la revendication 10, caractérisée en ce qu'elle contient une association de 1,136 % de monofluorophosphate de sodium, 0,773 % de fluorure de sodium, 15 % de xylitol, au sein d'un excipient convenable.

**Claims**

1. Compositions for the hygiene and health of the periodontal tissue of the gums in the form of a paste or gel characterised in that they contain a combination of two fluorinated salts, sodium monofluorophosphate combined with sodium fluoride, ammonium bifluoride combined with sodium fluoride, said combination having a fluorine ion concentration of between 200 mg and 2,000 mg per 100 g of composition, that is between 2,000 and 20,000 ppm, the fluorine ion concentration preferably being between 250 and 1,000 mg per 100 g of composition, that is between 2,500 and 10,000 ppm, xylitol in a concentration of 5 to 50 % by weight, based on the final composition, and preferably between 10 and 25 %, within a suitable excipient.

2. Compositions for the hygiene and health of the periodontal tissue of the gums in the form of a paste or gel according to claim 1, characterised in that they contain a combination of sodium monofluorophosphate and sodium fluoride having a fluorine ion concentration of 250 mg per 100 g of composition and xylitol in a concentration of 20 % by weight, based on the final composition, within a suitable excipient.

3. Compositions for the hygiene and health of the periodontal tissue of the gums in the form of a paste or gel according to claim 1, characterised in that they contain a combination of sodium monofluorophosphate and sodium fluoride having a fluorine ion concentration of 250 mg per 100 g of composition and xylitol in a concentration of 15 % by weight, based on the final composition, within a suitable excipient.

4. Compositions for the hygiene and health of the periodontal tissue of the gums in the form of a paste or gel according to claim 1, characterised in that they contain a combination of sodium monofluorophosphate and sodium fluoride having a fluorine ion concentration of 250 mg per 100 g of composition and xylitol in a concentration of 10 % by weight, based on the final composition, within a suitable excipient.

5. Compositions for the hygiene and health of the periodontal tissue of the gums in the form of a paste or gel characterised in that they contain 0.76 % of sodium monofluorophosphate, 0.33 % of sodium fluoride, 20 % of xylitol, within a suitable excipient.

6. Compositions for the hygiene and health of the periodontal tissue of the gums in the form of a paste or gel according to claim 1, characterised in that they contain a combination of ammonium bifluoride and sodium fluoride having a fluorine ion concentration of 250 mg per 100 g of composition and xylitol in a concentration of 20 % by weight, based on the final composition, within a suitable excipient.

7. Compositions for the hygiene and health of the periodontal tissue of the gums in the form of a paste or gel according to claim 1, characterised in that they contain a combination of ammonium bifluoride and sodium fluoride having a fluorine ion concentration of 250 mg per 100 g of composition and xylitol in a concentration of 15 % by weight, based on the final composition, within a suitable excipient.

8. Compositions for the hygiene and health of the periodontal tissue of the gums in the form of a paste or gel according to claim 1, characterised in that they contain a combination of sodium monofluorophosphate and sodium fluoride having an ion concentration of 500 mg per 100 g of composition and xylitol in a concentration of 20 % by weight, based on the final composition, within a suitable excipient.

9. Composition for the hygiene and health of the periodontal tissue of the gums in the form of a paste or gel according to claim 8, characterised in that it contains a combination of 0.800 % of sodium monofluorophosphate, and 0.873 % of sodium fluoride, 20 % of xylitol within a suitable excipient.

10. Compositions for the hygiene and health of the periodontal tissue of the gums in the form of a paste or gel according to claim 1, characterised in that they contain a combination of sodium monofluorophosphate and sodium fluoride having an ion concentration of 500 mg per 100 g of composition and xylitol in a concentration of 15 % by weight, based on the final composition, within a suitable excipient.

11. Composition for the hygiene and health of the periodontal tissue of the gums in the form of a paste or gel according to claim 10, characterised in that it contains a combination of 1.136 % of sodium monofluorophosphate, 0.773 % of sodium fluoride, 15 % of xylitol within a suitable excipient.

**Patentansprüche**

1. Mittel zur Hygiene und Gesunderhaltung des Parodontiums des Zahnfleisches und der Zähne in Form von Paste, Gel dadurch gekennzeichnet, daß sie eine Kombination von zwei Fluorsalzen, dem Natriummonofluorphosphat zusammen mit Natriumfluorid und dem Ammoniumbifluorid zusammen mit Natriumfluorid, enthalten, wobei die Kombination eine Konzentration an Fluorion zwischen 200 mg und 2 000 mg auf 100 g des Mittels aufweist, d. h. zwischen 2 000 und 20 000 ppm Fluorion, vorzugsweise zwischen 250 und 1 000 mg auf 100 g des Mittels, d. h. 2 500 bis 10 000 ppm, und Xylit in einer Konzentration von 5 bis 50 Gew.-%, bezogen auf das endgültige Mittel, und vorzugsweise zwischen 10 und 25 %, in einem geeigneten Excipiens.

2. Mittel zur Hygiene und Gesunderhaltung des Parodontiums des Zahnfleisches und der Zähne in Form von Paste, Gel nach Anspruch 1, dadurch gekennzeichnet, daß sie eine Kombination von Natriummonofluorphosphat und Natriumfluorid in einer Konzentration an Fluorion von 250 mg auf 100 g des Mittels und Xylit in einer Konzentration von 20 Gew.-%, bezogen auf das endgültige Mittel, in einem geeigneten Excipiens enthalten.

3. Mittel zur Hygiene und Gesunderhaltung des Parodontiums des Zahnfleisches und der Zähne in Form von Paste, Gel nach Anspruch 1, dadurch gekennzeichnet, daß sie eine Kombination von Natriummonofluorphosphat und Natriumfluorid in einer Konzentration an Fluorion von 250 mg auf 100 g des Mittels und Xylit in einer Konzentration von 15 Gew.-%, bezogen auf das endgültige Mittel, in einem geeigneten Excipiens enthalten.

4. Mittel zur Hygiene und Gesunderhaltung des Parodontiums des Zahnfleisches und der Zähne in Form von Paste, Gel nach Anspruch 1, dadurch gekennzeichnet, daß sie eine Kombination von Natriummonofluorphosphat und Natriumfluorid in einer Konzentration an Fluorion von 250 mg auf 100 g des Mittels und Xylit in einer Konzentration von 10 Gew.-%, bezogen auf das endgültige Mittel, in einem geeigneten Excipiens enthalten.

5. Mittel zur Hygiene und Gesunderhaltung des Parodontiums des Zahnfleisches und der Zähne in Form von Paste, Gel nach Anspruch 1, dadurch gekennzeichnet, daß es 0,76 % Natriummonofluorphosphat, 0,33 % Natriumfluorid, 20 % Xylit in einem geeigneten Excipiens enthält.

6. Mittel zur Hygiene und Gesunderhaltung des Parodontiums des Zahnfleisches und der Zähne in Form von Paste, Gel nach Anspruch 1, dadurch gekennzeichnet, daß sie eine Kombination von Ammoniumbifluorid und Natriumfluorid in einer Konzentration an Fluorion von 250 mg auf 100 g des Mittels und Xylit in einer Konzentration von 20 Gew.-%, bezogen auf das endgültige Mittel, in einem geeigneten Excipiens enthalten.

7. Mittel zur Hygiene und Gesunderhaltung des Parodontiums des Zahnfleisches und der Zähne in Form von Paste, Gel nach Anspruch 1, dadurch gekennzeichnet, daß sie eine Kombination von Ammoniumbifluorid und Natriumfluorid in einer Konzentration an Fluorion von 250 mg auf 100 g des Mittels und Xylit in einer Konzentration von 15 Gew.-%, bezogen auf das endgültige Mittel, in einem geeigneten Excipiens enthalten.

8. Mittel zur Hygiene und Gesunderhaltung des Parodontiums des Zahnfleisches und der Zähne in Form von Paste, Gel nach Anspruch 1, dadurch gekennzeichnet, daß sie eine Kombination von Natriummonofluorphosphat und Natriumfluorid in einer Konzentration an Fluorion von 500 mg auf 100 g des Mittels und Xylit in einer Konzentration von 20 Gew.-%, bezogen auf das endgültige Mittel, in einem geeigneten Excipiens enthalten.

9. Mittel zur Hygiene und Gesunderhaltung des Parodontiums des Zahnfleisches und der Zähne in Form von Paste, Gel nach Anspruch 1, dadurch gekennzeichnet, daß es eine Kombination von 0,800 % Natriummonofluorphosphat und 0,873 % Natriumfluorid und 20 % Xylit in einem geeigneten Excipiens enthält.

10. Mittel zur Hygiene und Gesunderhaltung des Parodontiums des Zahnfleisches und der Zähne in Form von Paste, Gel nach Anspruch 1, dadurch gekennzeichnet, daß sie eine Kombination von Natriummonofluorphosphat und Natriumfluorid in einer Konzentration an Fluorion von 500 mg auf 100 g des Mittels und Xylit in einer Konzentration von 15 Gew.-%, bezogen auf das endgültige Mittel, in einem geeigneten Excipiens enthalten.

11. Mittel zur Hygiene und Gesunderhaltung des Parodontiums des Zahnfleisches und der Zähne in Form von Paste, Gel nach Anspruch 10, dadurch gekennzeichnet, daß es eine Kombination von 1,136 % Natriummonofluorphosphat, 0,773 % Natriumfluorid und 15 % Xylit in einem geeigneten Excipiens enthält.